# EUROPEAN PATENT APPLICATION

(11) **EP 4 098 186 A1**
(43) Date of publication of application: **07.12.2022**
(21) Application number: 22177087.8
(22) Date of filing: 02.06.2022
(51) Int. Cl.: A61B 5/103, A47C 7/62, A61B 5/11

(54) **SITTING POSTURE MONITORING SYSTEM**

(30) Priority: 02.06.2021 ES 202130502
(71) Applicant: Universidad del Pais Vasco - Euskal Herriko Unibertsitatea (UPV/EHU), 48940 Leioa (ES)
(72) Inventor: Cabanes Axpe, Itziar, Leioa (ES); Mancisidor Barinagarrementeria, Aitziber, Leioa (ES); Vermander García, Patrick, Leioa (ES); Pérez Odriozola, Nerea, Leioa (ES); Brull Mesanza, Asier, Leioa (ES); Portillo Pérez, Eva, Leioa (ES); Zubizarreta Pico, Asier, Leioa (ES); Pérez, César, Leioa (ES)
(74) Representative: Balder IP Law, S.L.

(57) **Abstract**

The present invention relates to a system for monitoring sitting posture of a user, comprising a data acquisition module, a receiving module communicatively connected with the data acquisition module and a portable sensor device that can be coupled to a seat element comprising a seat portion and a backrest portion. The portable sensor device comprises eight pressure sensors embedded in specific positions of the seat portion and eight pressure sensors embedded in specific positions of the backrest portion. The sixteen sensors are operatively connected to the data acquisition module. The data collected by the postural monitoring system is displayed through a graphic interface for the processing and storage thereof.

## Description

### OBJECT OF THE INVENTION

The present invention belongs to the field of health in general, and more specifically, to the field of monitoring the sitting posture of users, especially those who spend a lot of time sitting for mobility or work reasons, among other reasons.

### BACKGROUND OF THE INVENTION

Existing postural monitoring technological solutions are generally classified into three large groups, depending on how invasive they are for the user: vision sensors, wearable sensors and pressure sensors. Currently, the postural monitoring products that stand out the most on the market are those belonging to the groups of wearable and pressure sensors.

The group of technological solutions related to wearable sensors is characterised in that they are incorporated or attached to clothing or to the body of the user itself, enabling information to be collected throughout the day on the physical activity carried out by the user who wears them continuously. Along this line, the use of inertial measurement units (IMU) or accelerometers is frequent, which enable the estimation of the speed and orientation of those points wherein said sensors are located. Document ES1167834 describes a device for monitoring the human body that uses wearable sensors located in different parts of the human body. These sensors emit signals that are collected by receivers that are programmed to quantify the intensity of the emitted signal, enabling the distance between sensors and the location thereof to be inferred. Document WO2011124732 describes the use of accelerometer sensors placed along the human body and document ES2364172 also describes the use of accelerometers in conjunction with gyroscopes and beats.

These wearable sensors are generally small in size and easy to implement. Their small dimensions enable them to be incorporated into clothing, thus creating highly portable devices. However, this portability is linked to an increase in the degree of invasion in users. These are devices made up of sensors that, being attached to the clothing itself or coupled to the body of the user, generate discomfort in those users wearing them. Not only that, the friction between the sensors with the body itself can be a source of discomfort for the user and of interference for the sensors which alter the measurements collected.

The second group of postural monitoring devices is made up of sensors based on vision technologies. These sensors are based on the acquisition and capture of images through cameras located in the surroundings, to later, and using artificial vision algorithms, extract the relevant information from the captured image. Among the cameras used, the Kinect camera stands out above the rest. Although these types of sensors avoid the intrinsic discomfort of wearable sensors, they are more complex to install as they have to be placed in a very specific location. This location is highly relevant, since the data collected can be altered by changes in luminosity, as well as possible obstacles that stand between the camera and the monitored user. In addition, these devices are more commonly used to monitor the postures of users while standing, and not so much for the sitting posture, since the seat itself can act as an obstacle. Finally, when using vision cameras, more information is collected than is necessary for postural tracking, which can cause privacy issues.

The third group of postural monitoring devices are pressure sensors that are placed on rigid or semi-rigid contact surfaces and not on the body of the user and that are configured to extract information on the pressure exerted on the points where they are located. Since they are placed on an external surface, they minimise the degree of intrusion on the user, while ensuring great portability and that the use thereof does not interfere with the postural development of the people they monitor. Unlike vision systems, pressure sensors only record pressure distributions, thus ensuring user privacy. Within this group, capacitive and resistive transduction sensors stand out.

The most common manner in which capacitive transduction sensors are implemented for postural measurement is in textile sensor format. These sensors are generally sensitive to small changes, so that they enable low pressure ranges to be measured with great accuracy. In addition, to determine postural changes, the exact pressures at each point are not to be known, but rather the variation of the measurements between one posture and another. This, linked to the fact that capacitive transduction sensors are costly, means that they are not widely used for postural detection applications.

The most widely used sensors for postural monitoring are resistive transduction sensors. The principle of operation of these sensors consists in the measurement of changes in voltage due to changes in the resistivity of the sensors. Among the advantages of these sensors are the low price thereof and their flexibility to adapt to different sensitivities and pressure ranges. However, the most common form in which these sensors are presented is in the form of a mesh. For manufacturing these meshes, smart fabrics or E-textiles can be used that record electric signals produced by the compression of the fibres. Otherwise, a combination of resistive sensors can be made, grouping them into rows and columns, creating a mesh of sensors arranged in the form on an array. In general, due to the high number of measurement units that make them up, they have a very high economic cost. In addition, this high number of sensors means that, if they are being used wirelessly, the use time thereof is limited.

Currently there are commercial pressure sensors in the form of a mesh. Thus, document WO2014113897 describes a system for preventing ulna ulcers or bedsores consisting of a set of pressure, temperature and humidity sensors that are only integrated in the seat of the chair. Document WO2016053398A1 discloses a cushion made up of pressure and fibre optic sensors for postural monitoring of the user that only rests on the seat (not on the backrest) and that also monitors vital signs, such as heart rate or respiration rate. Focussing their attention on the detection of falls, the device of WO2014188187 incorporates two pressure sensors that are only located in the backrest, monitoring being focussed on the reclining of the back. However, the inventions of the cited documents exhibit a series of limitations or shortcomings. In the first place, they have a reduced number of generic sensors, which are only located on the seat or the backrest. Therefore, information regarding the status of the seat-backrest assembly of the user is lost, reducing the number of postures to be monitored. This limitation in terms of the number of postures is clearly reflected in document ES1227019 wherein only the monitoring of the meditation posture is sought.

Finally, many of the existing solutions for monitoring sitting posture do not have a simple graphical interface that enables the data collected to be transmitted in a clear and intuitive manner, or they only enable the data that is being collected at the moment to be viewed, without the possibility of applying subsequent analysis techniques.

### DESCRIPTION OF THE INVENTION

A first object of the present invention is a system for monitoring the sitting posture of a user according to that which is described in claim 1. Particular embodiments of the invention are described in the dependent claims.

The system for monitoring the sitting posture of users comprises a data acquisition module, a receiving module communicatively connected with the data acquisition module and a portable sensor device that is configured to be coupled to a seat element. The seat element could be a chair, wheelchair, armchair, sofa, or the like, which has both a seat surface and a backrest surface. The portable sensor device may comprise rubber bands, clips, Velcro^{®}, or any other coupling mechanism for coupling to the seat element. Said portable sensor device in turn comprises a seat portion and a backrest portion. The seat portion is adapted to be coupled to the seat surface of the seat element and the backrest portion is adapted to be coupled to the backrest surface of the seat element. As used herein, the term "portable" means that the sensor device is easy or simple to move, transfer, transport, haul, bring or carry. Therefore, the portable sensor device enables the transfer and coupling/uncoupling thereof to different seat elements located in different places.

When the portable sensor device is coupled or mounted on the seat element, the seat portion defines an XY plane in correspondence with the seat surface of the seat element and the backrest portion defines a YZ plane in correspondence with the backrest surface of the seat element. The seat portion and the backrest portion define a contact line in correspondence with the Y axis so that a coordinate origin is defined at a midpoint of the width of the seat and backrest portion. Although the surface of both the seat portion and the backrest portion may be of variable width, the coordinate origin will be defined at the midpoint of this width. Thus, by way of example, if the width is 50 cm, the coordinate origin is located at 25 cm measured from the lateral surface of any of the two portions. If the seat and backrest portions had different widths, a coordinate origin could be established for each of the portions considering the respective widths thereof. Both the seat portion and the backrest portion will preferably have a square or rectangular shape, although they may also have other shapes such as oval or circular and even irregular shapes. As defined herein, the line of contact between the seat portion and the backrest portion that is located in correspondence with the Y axis relates to the line that would define the inner edges of the seat and backrest portions when they make contact (or the projection of the same, if they do not come into contact), i.e., the line that would be defined by the edges of the portions that are located in correspondence with the join between the backrest surface and the seat surface of the seat element. If the inner edges of the seat and backrest portions have curved shapes, the contact line can correspond to the tangential line to said curved edges that are located in correspondence with the junction between the backrest surface and the seat surface of the seat element.

The portable sensor device comprises eight pressure sensors (A1-8) embedded in the seat portion and eight pressure sensors (R9-16) embedded in the backrest portion. The sixteen sensors are operatively connected to the data acquisition module. Both the backrest portion and the seat portion can be formed by a sheet of rigid or semi-rigid material on which the pressure sensors are fixed and said sheet can be covered with a textile material.

In some embodiments, the data acquisition module can be directly connected by means of respective cables (USB, microUSB or any other known wired communication interface) to the corresponding pressure sensors of each of the portions of the portable sensor device. In said embodiments the data acquisition module will be coupled to the seat element or placed in proximity of the same during the monitoring operation. Alternatively, each of the portions of the portable sensor device can incorporate a transmitter that is connected to the corresponding pressure sensors and that is configured to send the captured measurements to a remote data acquisition module via a receiver integrated in the data acquisition module itself. In said embodiments, the transmitter can be a GPS, GPRS, 3G, 4G or 5G antenna, a WIFI, RFID or Bluetooth communications module, etc. The data acquisition module can also integrate the electronics associated to the pressure sensors and can be powered by an external power supply. In turn, the receiver module can be operatively communicated with the data acquisition module through a wireless communication interface such as Bluetooth, RFID, USB, GPS, GPRS, 2G, 4G, 5G, LTE, CatM, NB loT, Sigfox, Lora or other communication standards, preferably in the form of an input/output subsystem, including the corresponding antennas, transceivers, controllers, connectors, ports, etc. Said receiver module can be integrated into a user computing device such as a desktop or laptop computer, a mobile phone or a tablet, among others, from which, through a graphical interface embedded in the receiver module itself or in the computing device, the data captured by the pressure sensors can be processed to analyse the sitting posture of the user being monitored.

The pressure sensors of the seat portion are located in the following coordinate ranges (X,Y,Z): a first seat sensor (A1) in the range X=[310-350],Y=[-100- -140], a second seat sensor (A2) in the range X=[180-220],Y=[-100- -140], a third seat sensor (A3) in the range X=[100-140 ],Y=[-60- -100], a fourth seat sensor (A4) in the range X=[180-220], Y=0, a fifth seat sensor (A5) in the range X=[50-90], Y=0, a sixth seat sensor (A6) in the range X=[310-350],Y=[100-140], a seventh seat sensor (A7) in the range X=[180-220],Y=[100-140] and an eighth seat sensor (A8) in the range X=[100-140],Y=[60-100]. The values of the coordinates are measured in millimetres from the previously defined coordinate origin. Since all of the pressure sensors of the seat portion are located in the XY plane defined by the seat surface of the seat element, the Z coordinate of all of them is 0.

As for the pressure sensors of the backrest portion, they are located at the following coordinates (X,Y,Z): a first backrest sensor (R9) in the range Y=[-100--140], Z= [120-160], a second backrest sensor (R10) in the range Y=[-100- -140], Z=[240-280], a third backrest sensor (R11) in the range Y=[-40- -80], Z=[180-220], a fourth backrest sensor (R12) in the range Y=0, Z=[90-120], a fifth backrest sensor (R13) in the range Y=0, Z=[310-350], a sixth backrest sensor (R14) in the range Y=[40-80], Z=[180-220], a seventh backrest sensor (R15) in the range Y=[100-140], Z=[120-160] and an eighth backrest sensor (R16) in the range Y=[100-140], Z=[240-280]. The values of the coordinates are measured in millimetres from the previously defined coordinate origin. Since all the pressure sensors of the backrest portion are located in the YZ plane defined by the backrest surface of the seat element, the X coordinate of all of them is 0.

In some embodiments, the value of the X-axis coordinate is the same for the following set of pairs of pressure sensors of the seat portion: the third seat sensor and the eighth seat sensor, the second seat sensor and the seventh seat sensor and the first seat sensor and the sixth seat sensor. In this way, the pressure sensors of said pairs are located symmetrically with respect to the plane of symmetry XZ.

In some embodiments, the value of the Z-axis coordinate is the same for the next set of pairs of pressure sensors of the backrest portion: the first backrest sensor and the seventh backrest sensor, the third backrest sensor and the sixth backrest sensor and the second backrest sensor and the eighth backrest sensor. In this way, the pressure sensors of said pairs are located at the same height with respect to the seat surface of the seat element and therefore monitor points on the back of the user that are located at the same height.

In some embodiments, the value of the Y-axis coordinate is the same for the following set of pairs of the pressure sensors: the first backrest sensor and the second backrest sensor, the seventh backrest sensor and the eighth backrest sensor, the first seat sensor and the second seat sensor and the sixth seat sensor and the seventh seat sensor. In this way the pressure sensors of said pairs are located symmetrically with respect to the plane of symmetry XZ.

Preferably, the pressure sensors of the seat portion are located at the following coordinates: the first seat sensor at X=330,Y=-120, the second seat sensor at X=200,Y=-120, the third seat sensor at X=120,Y=-80, the fourth seat sensor at X=200, Y=0, the fifth seat sensor at X=70, Y=0, the sixth seat sensor at X=330,Y=120, the seventh seat sensor at X=200,Y=120 and the eighth seat sensor at X=120,Y=80. These coordinate values are measured in millimetres with respect to the previously defined coordinate origin. Since all of the pressure sensors of the seat portion are located in the XY plane defined by the seat surface of the seat element, the Z coordinate of all of them is 0. The location of the pressure sensors in these positions in the seat portion optimises and improves the monitoring of the sitting position of the user, and more specifically of the position of the thighs and buttocks of the user when seated.

Preferably, the pressure sensors of the backrest portion are located at the following coordinates: the first backrest sensor at Y=-120, Z=140, the second backrest sensor at Y=-120, Z=260, the third backrest sensor at Y=-60, Z=200, the fourth backrest sensor at Y=0, Z=110, the fifth backrest sensor at Y=0, Z=330, the sixth backrest sensor at Y=60, Z=200, the seventh backrest sensor at Y=120, Z=140 and the eighth backrest sensor at Y=120, Z=260. These coordinate values are measured in millimetres with respect to the previously defined coordinate origin. Since all the pressure sensors of the backrest portion are located in the YZ plane defined by the backrest surface of the seat element, the X coordinate of all of them is 0. The location of the pressure sensors in these positions in the backrest portion optimises and improves the monitoring of the sitting position of the user, and more specifically of the position of the back of the user when seated.

In some embodiments, the first seat sensor and the sixth seat sensor are configured to monitor the biceps femoris of the user, the second seat sensor and the seventh seat sensor are configured to monitor the gluteus maximus of the user, the third seat sensor and the eighth seat sensor are configured to monitor the ischia of the user, the fourth seat sensor is configured to monitor the perineum of the user, and the fifth seat sensor is configured to monitor the sacrum of the user. In this way, the least possible number of points corresponding to specific muscles and bones of the user are monitored, which allow a correct characterisation of the sitting position of the user on the seat surface of the seat element on which it rests.

In some embodiments, the first backrest sensor and the seventh backrest sensor are configured to monitor lateral movements of the lumbar region of the user, the third backrest sensor and the sixth backrest sensor are configured to monitor the lower thoracic region of the user, the second backrest sensor and the eighth backrest sensor are configured to monitor the middle thoracic region of the user, the fourth backrest sensor is configured to monitor the central lumbar region of the user, and the fifth backrest sensor is configured to monitor the upper-central thoracic region. In this way, the least possible number of points corresponding to specific muscles and bones of the user are monitored, which allow the sitting position of the user on the backrest surface of the seat element on which it rests to be appropriately characterised.

In some embodiments, the 16 pressure sensors are selected from resistive pressure sensors, capacitive pressure sensors, and a combination of both. Preferably, the 16 pressure sensors are resistive pressure sensors. These resistive pressure sensors have a lower price and exhibit higher adaptability to different sensitivities and pressure ranges than capacitive pressure sensors.

In some embodiments, the seat and backrest portions are symmetrical with respect to the plane of symmetry XZ located in correspondence with the coordinate origin.

In some embodiments, the receiver module is communicatively connected with data storage means (for example, a RAM memory) wherein the measurements captured by the pressure sensors are stored, and from where they can be retrieved and processed for personalised monitoring of each user.

In addition, through the graphical interface incorporated in the receiving module or in the user computing device wherein the receiving module is integrated, said information can be viewed in real time and information on the sitting posture can be generated to be displayed on the device of the user, and send a notification to the device of the user.

Thus, the system for monitoring sitting posture of a user is made up of 16 pressure sensors discreetly located in correspondence with key points of the anatomy of the user, enabling a continuous real-time monitoring of the sitting posture of the user to be performed. The system described herein makes it possible to characterise a minimum of 12 different sitting postures adopted by the user placed on top of it, at an economical price and with a time greater than 24 hours. Unlike other existing solutions where attention is solely focussed on the seat, the system object of the present invention comprises pressure sensors located at key points on both the seat and backrest surfaces. With the arrangement of the pressure sensors described, it is possible to reduce the total number of sensors used to characterise the sitting posture of the user, but without this resulting in a significant loss of postural information. In this way, it is possible to reduce the cost of the system compared to other existing systems for monitoring sitting posture. In addition, the fact of using a smaller number of sensors makes it possible to reduce the energy consumption of the system as a whole, and of the portable sensor device in particular, enabling the continuous use thereof for a longer period of time. Moreover, a smaller number of data to be monitored allows the subsequent analysis and processing thereof to be simplified. In this way, unlike other devices, such as sensor meshes, wherein the capture of postural information is based on the gathering of a large number of measurement points, without taking into account the relevance of the same for postural monitoring, the monitoring system described herein focusses on monitoring only the relevant points, simplifying the data capture and processing process. Additionally, the graphical interface not only shows and enables the data collected to be analysed, but it will also enable relevant indicators of postural assessment to be inferred, providing additional information of high added value to both the user and the health personnel, thus managing to avoid abnormal postures, long-term muscle problems and early fall detection.

### BRIEF DESCRIPTION OF THE FIGURES

Figure 1 shows a block diagram of a system for monitoring sitting posture of users, according to a particular embodiment of the invention.
Figure 2 shows a perspective view of the seat and backrest of a chair to which the portable sensor device has been coupled, according to a particular embodiment of the invention. Figure 2 also shows a plan view of the seat portion and the backrest portion of the portable sensor device.
Figure 3 shows a plan view of the seat portion, indicating the ranges for the coordinates that define the positions of the seat pressure sensors with respect to the coordinate origin.
Figure 4 shows a plan view of the backrest portion, indicating the ranges for the coordinates that define the positions of the backrest pressure sensors with respect to the coordinate origin.

### PREFERRED EMBODIMENT OF THE INVENTION

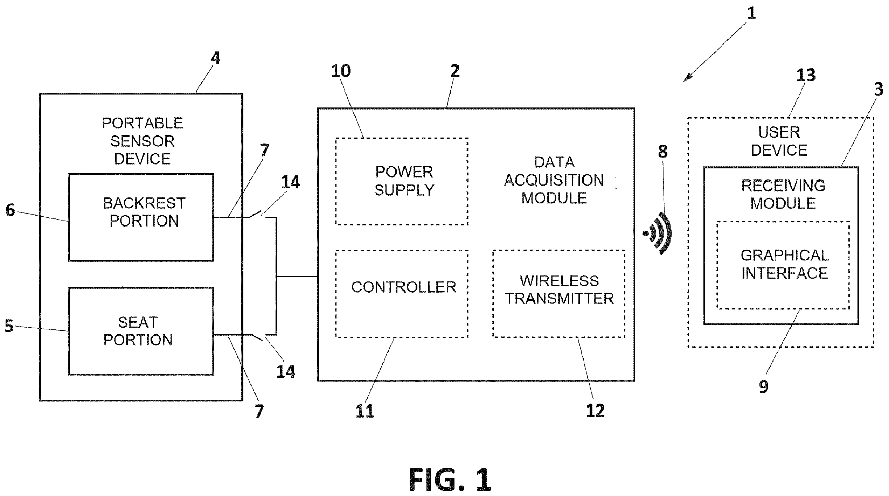

Figure 1 shows a block diagram of a system (1) for monitoring sitting posture of users, according to a particular embodiment of the invention.

The portable sensor device (4) is formed by a seat portion (5) and a backrest portion (6) wherein each one of said portions integrates 8 resistive pressure sensors (not shown in this figure), which are fixed at specific points on respective sheets or plates of rigid or semi-rigid material. Preferably, rigid materials will be chosen, and the sheets or plates may be curved or flat to better adapt to the surface on which they rest and to adapt to the shape of the body of the user. In addition, the aforementioned sheets or plates, and therefore the seat and backrest portions (5,6) will have dimensions that are compatible with a standard chair, although said dimensions may be adapted depending on the specific application. These plates or sheets are covered with a padded textile material such that the seat and backrest portions exhibit contact surfaces with the user that are padded in the form of a cushion.

Each one of the pressure sensors of the seat and backrest portions (5,6) will be connected by means of two cables, one for power supply (at 5 V) and another for grounding to one of the analogue pins of the data acquisition module (2), and on the other hand to a resistor, forming a voltage divider (not shown). The value of the resistance can be variable, preferably being of a value of 10 kilo ohms. In the same way, this resistance can be joined both to ground and to the power supply, preferably external. In the first case, there is an increase in the signal measured from the analogue pin with an increase in pressure on the sensor, and in the second, a decrease from an initial voltage of 5 V.

The cables coming out of the sensors will be grouped into two groups of eight cables each, a first group (7) corresponding to the seat and a second group (7) corresponding to the backrest, and will be incorporated into two connectors or switches (14) enabling a quick and easy coupling and uncoupling of the pressure sensors, and therefore of the seat and backrest portions (5,6) with the data acquisition module (2). The data acquisition module (2) integrates a controller (11) comprising the software and hardware that are required to implement the processing of the data received from the pressure sensors and to execute the required functionalities in terms of calculation capacity to execute automation and/or control functions. Said data acquisition module (2) also integrates a memory for the temporary storage of the data received from the sensors. The controller (11) can be a microprocessor, a central processing unit (CPU), a programmable logic controller, or other similar device capable of carrying out the functionalities described herein.

The data acquisition module (2) also comprises a power supply system (10) that can be made up, for example, of an internal battery or an external lithium, LiPo, lithium-ion battery or similar connected to the acquisition module (2) through a USB input. Preferably, the battery will have a capacity greater than 2200 mAh. Alternatively, the data acquisition module (2) can be powered via a USB connection with the computer, telephone or any other electronic device (13) of the user wherein the receiver module (3) is housed and, therefore, where the graphic monitoring interface (9) is being executed.

The data acquisition module (2) also comprises a wireless transmitter (12) configured to connect to the user device, for example, a computer, where the graphical interface (9) will be housed by means of a wireless communication protocol. This connection (8) will be done using either WiFi or Bluetooth, the latter being preferable.

In order to provide rigidity to the system and provide greater security to the electronic connections, the data acquisition module (2) can be integrated into a protective casing (not shown).

The data acquisition module (2) receives the data from the portable sensor device (4) and the controller (11) determines the pressure values in each sensor and for each instant from the received data. These pressure values are transmitted by means of the wireless transmitter (12) to the user device (13) where they are stored in a text file. The graphical interface (9) will access the text files, and by means of an appropriate graphical environment, for example, Matlab type, it will allow working with the acquired data, viewing them, saving them, or processing or analysing them.

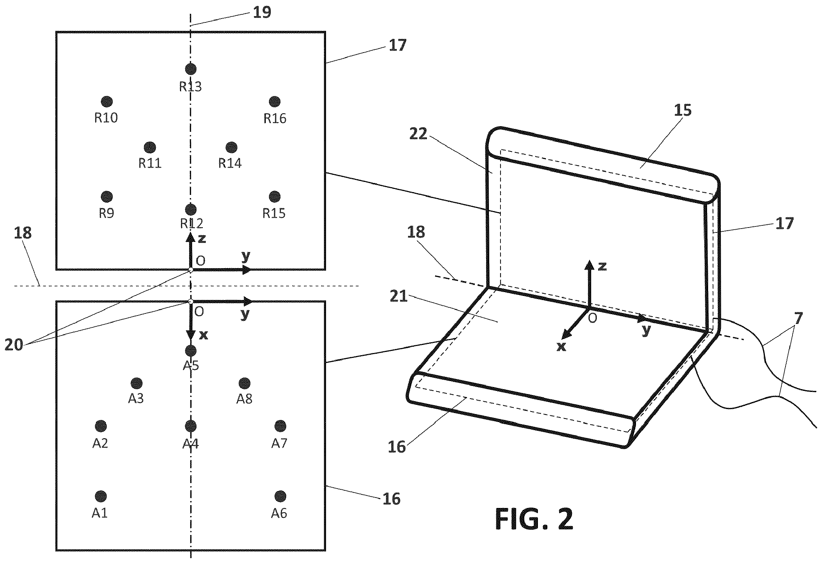

Figure 2 shows a perspective view of the seat and backrest of a chair (15) (without the legs) to which the portable sensor device has been attached, according to a particular embodiment of the invention, and more specifically, the seat portion (16) and the backrest portion (17) of the portable sensor device. Figure 2 also shows a plan view of the seat portion (16) and the backrest portion (17) of the portable sensor device.

To define the location of the pressure sensors (A1-8, R9-16), the central point of the contact line (18) between the seat portion (16) and the backrest portion (17) of the chair (15) is taken as coordinate origin (20). Said seat and backrest portions (16,17) are coupled on the seat and backrest surfaces (21,22) of the chair (15), respectively. Although the seat and backrest portions (16,17) can be of variable width, the midpoint of this width is taken as the coordinate origin (20). Thus, by way of example, if the width is 50 cm, the coordinate origin is located at 25 cm measured from the lateral edge of any of the portions (16,17). By way of identification, the 8 sensors of the seat portion have been named using the letter 'A', (from A1 to A8), and the sensors of the backrest portion using the letter 'R' (from R9 to R16).

This coordinate origin (20) will delimit an XZ plane of symmetry (19) for the pressure sensors (A1-8, R9-16) both in the seat portion (16) and in the backrest portion (17). In this way, maintaining symmetry with respect to the XZ plane (19) at all times, for each one of the sensors (A1-8, R9-16) coordinates are established, within ranges, with respect to the coordinate origin (20) to delimit the location thereof such that an optimal monitoring of the sitting position of the user is guaranteed. Thus, in the seat portion (16), the X-axis coordinate has to be the same (within the given range) for the following set of pairs of sensors: the first seat sensor (A1) and the sixth seat sensor (A6), the second seat sensor (A2) and the seventh seat sensor (A7) and the third seat sensor (A3) and the eighth seat sensor (A8). Moreover, in the backrest portion (17), the Z-axis coordinate has to be the same (within the given range) for the next set of pairs of sensors: the first backrest sensor (R9) and the seventh sensor backrest sensor (R15), the third backrest sensor (R11) and the sixth backrest sensor (R14) and the second backrest sensor (R10) and the eighth backrest sensor (R16). Likewise, it is desirable that the Y axis coordinate is the same (within the specific range) between the following pairs of sensors: the first backrest sensor (R9) and the second backrest sensor (R10), the seventh backrest sensor (R15) and the eighth backrest sensor (R16), the first seat sensor (A1) and the second seat sensor (A2), and the sixth seat sensor (A6) and the seventh seat sensor (A7).

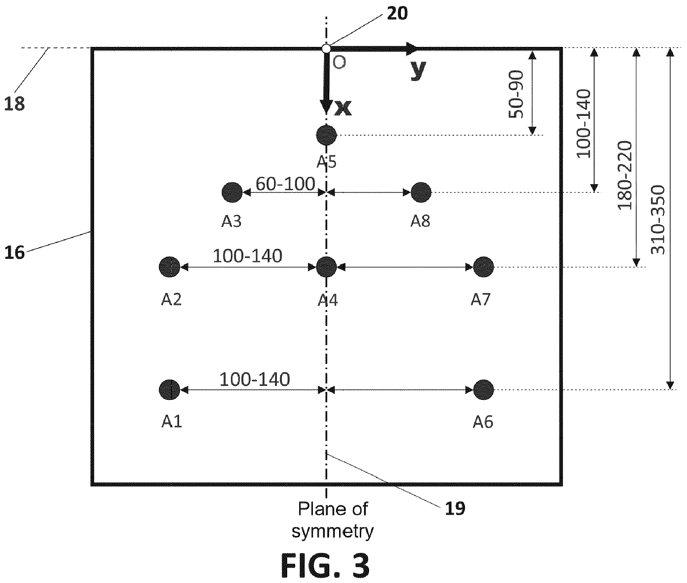

Figure 3 shows a plan view of the seat portion (16), indicating the ranges for the coordinates that define the positions of the seat pressure sensors (A1-8) with respect to the coordinate origin (20). These ranges determine the maximum and minimum distances, in millimetres, of the seat pressure sensors (A1-8) with respect to the coordinate origin (20)

All the pressure sensors (A1-8) of the seat portion (16) are positioned in the XY plane defined in Figure 2, so the location of each sensor is defined based on those two coordinates since the coordinate thereof on the Z axis is zero. Two sensors are placed on the X axis: the fifth seat sensor (A5), for monitoring the sacrum, located at the coordinates (X = 50-90, Y = 0) and the fourth seat sensor (A4) located at the coordinates (X = 180-220, Y = 0), for monitoring the perineum. The first seat sensor (A1) and the sixth seat sensor (A6) are located at a distance in the X axis between 310 and 350 mm, and are offset between 100 and 140 mm (the first seat sensor (A1) in the negative direction and the sixth seat sensor (A6) in the positive direction of the axis Y) with respect to the XZ plane (19) of symmetry of the seat portion (16). The second seat sensor (A2) and the seventh seat sensor (A7) are located at a distance in the X axis between 180 and 220 mm, and are offset between 100 and 140 mm (the second seat sensor (A2) in the negative direction and the seventh seat sensor (A7) in the positive direction of the axis Y) with respect to the XZ plane (19) of symmetry of the seat portion (16).

Both the set formed by the first seat sensor (A1) and the sixth seat sensor (A6) and the set formed by the second seat sensor (A2) and the seventh seat sensor (A7) serve to monitor the thighs, using the first set to track the biceps femoris, and the second set to monitor the gluteus maximus. Finally, the third seat sensor (A3) and the eighth seat sensor (A8) are located at a distance of between 100 and 140 in the direction of the X axis, separated between 60 and 100 mm with respect to the XZ plane (19) of symmetry (negative and positive direction respectively with respect to the Y axis). In this way, said third seat sensor (A3) and eighth seat sensor (A8) are configured to monitor the ischia of the user.

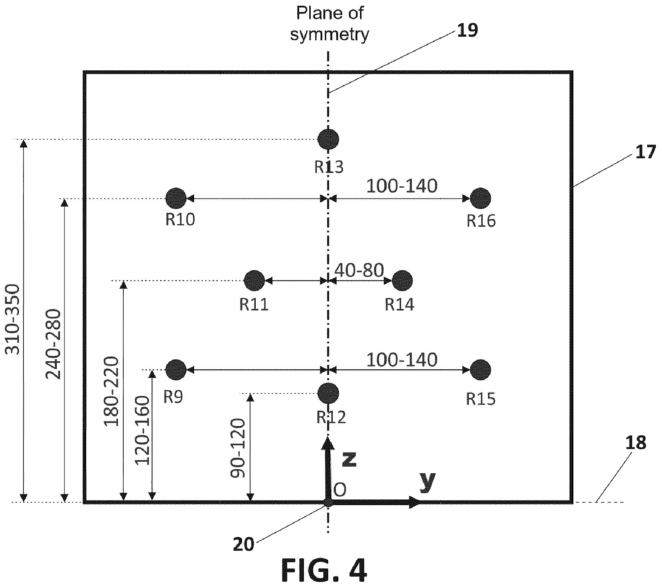

Figure 4 shows a plan view of the backrest portion (17), indicating the ranges for the coordinates that define the positions of the backrest pressure sensors (R9-16) with respect to the coordinate origin (20). These ranges determine the maximum and minimum distances, in millimetres, of the backrest pressure sensors (R9-16) with respect to the coordinate origin (20)

All the pressure sensors of the backrest are positioned in the ZY plane defined in figure2, so the location of each sensor is defined based on those two coordinates since the coordinate thereof on the Z axis is zero. The following are located on the Z axis: the fourth backrest sensor (R12) at a distance of between 90 and 120 mm from the coordinate origin (20), to monitor the central lumbar area of the back of the user, and the fifth backrest sensor (R13) at a distance between 310 and 350 mm from the coordinate origin (20), to monitor the upper-central thoracic region (between vertebrae T1 and T4). Both sensors have a coordinate Y=0.

The first backrest sensor (R9) and the seventh backrest sensor (R15), both at a distance on the Z axis from the coordinate origin (20) between 120 and 160 mm, are located at a distance between 100 and 140 mm in the direction of the Y axis (negative and positive direction, respectively). These sensors (R9, R15) serve to monitor lateral movements in the lumbar area of the back of the user. The second backrest sensor (R10) and the eighth backrest sensor (R16), maintain a distance in the Y-axis direction of between 100 and 140 mm, but are located between 240 and 280 mm in the Z axis. These sensors (R10, R16) are configured to monitor the mid-thoracic area of the back (between the T5 and T8 vertebrae).

Finally, the third backrest sensor (R11) and the sixth backrest sensor (R14), located at a distance of between 180 and 220 mm in the direction of the Z axis, maintain a separation with respect to the XZ plane (19) of symmetry of the backrest portion (17) of between 40 and 80 mm in the direction of the Y axis (negative and positive direction, respectively). These sensors (R11, R14) are responsible for monitoring the lower thoracic region of the back of the user (between the T9 and T12 vertebrae).

As a summary of the locations of the pressure sensors (A1-8, R9-16) described in figures 3 and 4, the following table is illustrated wherein the different ranges for each of the sensors (A1-8, R9-16), in the X, Y and Z coordinates, as well as coordinates that delimit a preferred location of said sensors (A1-8, R9-16) in the seat and backrest portions (16,17).

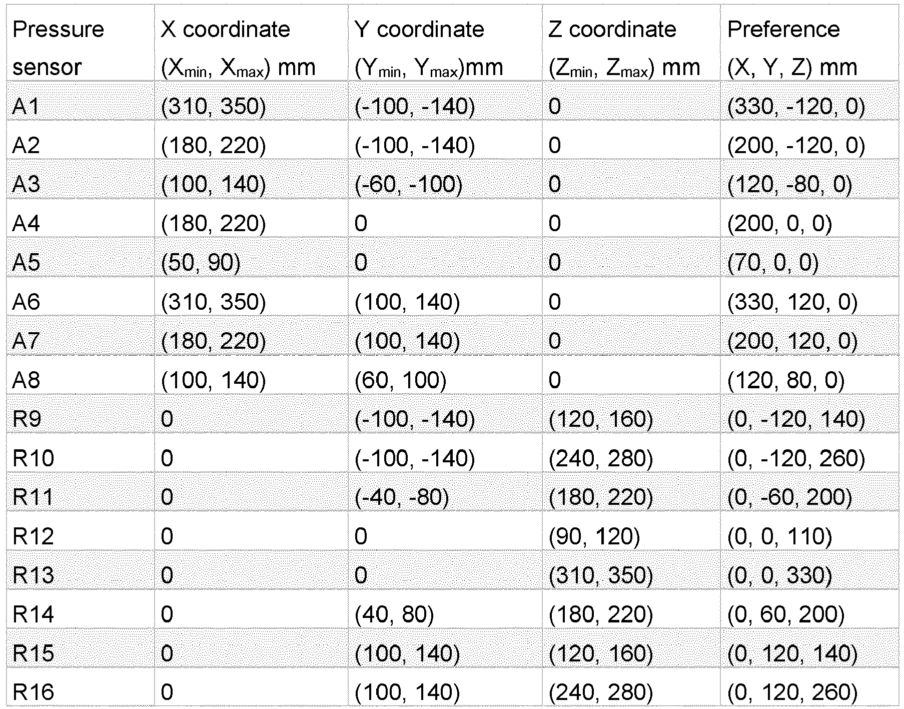

## Claims

1. A system for monitoring sitting posture (1) of a user, comprising:
a data acquisition module (2);
a receiver module (3) communicatively connected with the data acquisition module (2); and
a portable sensor device (4) comprising a seat portion (5) and a backrest portion (6), wherein the portable sensor device (4) is configured to be coupled to a seat element (15);
wherein, when the portable sensor device (4) is coupled to the seat element (15), the seat portion (5) defines an XY plane in correspondence with a seat surface (16) of the seat element (15) and the backrest portion (6) defines a YZ plane in correspondence with a backrest surface (17) of the seat element (15) and wherein the seat portion (5) and the backrest portion (6) define a contact line (18) in correspondence with the Y axis such that a coordinate origin (20) is defined at a midpoint of the width of the seat portion (5) and the backrest portion (6);
**characterised in that** the portable sensor device (4) comprises eight pressure sensors (A1-8) embedded in the seat portion (5) and eight pressure sensors (R9-16) embedded in the backrest portion (6), wherein the sixteen sensors (A1-8, R9-16) are operatively connected to the data acquisition module (2);
wherein the pressure sensors (A1-8) of the seat portion (5) are located at the following coordinates (X, Y, Z), in millimetres, with respect to the coordinate origin (20):
a first seat sensor (A1) at ([310-350],[-100- -140],0), a second seat sensor (A2) at ([180-220],[-100- -140],0), a third seat sensor (A3) at ([100-140],[-60- - 100],0), a fourth seat sensor (A4) at ([180-220],0,0), a fifth seat sensor (A5) at ([50-90],0,0), a sixth seat sensor (A6) at ([310-350],[100-140],0), a seventh seat sensor (A7) at ([180-220],[100-140],0) and an eighth seat sensor (A8) at ([100-140],[60-100],0); and
wherein the pressure sensors (R9-16) of the seat portion (6) are located at the following coordinates (X, Y, Z), in millimetres, with respect to the coordinate origin (20):
a first backrest sensor (R9) at (0,[-100- -140],[120-160]), a second backrest sensor (R10) at (0,[-100- -140],[240-280]), a third backrest sensor (R11) at (0,[-40- -80],[180-220]), a fourth backrest sensor (R12) at (0,0,[90-120]), a fifth backrest sensor (R13) at (0,0,[310-350]), a sixth backrest sensor (R14) at (0,[40-80],[180-220]), a seventh backrest sensor (R15) at (0,[100-140],[120-160]) and an eighth backrest sensor (R16) at (0,[100-140],[240-280]).

2. The system (1) of claim 1, wherein the value of the coordinate on the X axis is the same for the following set of pairs of pressure sensors of the seat portion (5): the third seat sensor (A3) and the eighth seat sensor (A8), the second seat sensor (A2) and the seventh seat sensor (A7), and the first seat sensor (A1) and the sixth seat sensor (A6).

3. The system (1) of claim 1 or 2, wherein the value of the coordinate on the Z axis is the same for the following set of pairs of pressure sensors of the backrest portion (6): the first backrest sensor ( R9) and the seventh backrest sensor (R15), the third backrest sensor (R11) and the sixth backrest sensor (R14) and the second backrest sensor (R10) and the eighth backrest sensor (R16).

4. The system (1) of any one of the preceding claims, wherein the value of the coordinate on the Y axis is the same for the following set of pairs of pressure sensors: the first backrest sensor (R9) and the second backrest sensor (R10), the seventh backrest sensor (R15) and the eighth backrest sensor (R16), the first seat sensor (A1) and the second seat sensor (A2) and the sixth seat sensor (A6) and the seventh seat sensor (A7).

5. The system (1) of any one of the preceding claims, wherein the pressure sensors (A1-8) of the seat portion (5) are located on the following coordinates (X, Y, Z), in millimetres, with respect to the coordinate origin (20):
the first seat sensor (A1) at (330,-120,0), the second seat sensor (A2) at (200,-120,0), the third seat sensor (A3) at (120,-80,0), the fourth seat sensor (A4) at (200,0,0), the fifth seat sensor (A5) at (70,0,0), the sixth seat sensor (A6) at (330,120,0), the seventh seat sensor (A7) at (200,120,0), and the eighth seat sensor (A8) at (120,80,0).

6. The system (1) of any one of the preceding claims, wherein the pressure sensors (R9-16) of the backrest portion (6) are located at the following coordinates, in millimetres, with respect to the coordinate origin (20):
the first backrest sensor (R9) at (0,-120,140), the second backrest sensor (R10) at (0,-120,260), the third backrest sensor (R11) at (0,-60,200), the fourth backrest sensor (R12) at (0,0,110), the fifth backrest sensor (R13) at (0,0,330), the sixth backrest sensor (R14) at (0,60,200), the seventh backrest sensor (R15 ) at (0,120,140) and the eighth backrest sensor (R16) at (0,120,260).

7. The system (1) of any one of the preceding claims, wherein the first seat sensor (A1) and the sixth seat sensor (A6) are configured to monitor the biceps femoris of the user, the second seat sensor (A2) and the seventh seat sensor (A7) are configured to monitor the gluteus maximus of the user, the third seat sensor (A3) and the eighth seat sensor (A8) are configured to monitor the ischia of the user, the fourth seat sensor (A4) is configured to monitor the perineum of the user and the fifth seat sensor (A5) is configured to monitor the sacrum of the user.

8. The system (1) of any one of the preceding claims, wherein the first backrest sensor (R9) and the seventh backrest sensor (R15) are configured to monitor lateral movements of the lumbar region of the user, the third backrest sensor (R11) and the sixth backrest sensor (R14) are configured to monitor the lower thoracic region of the user, the second backrest sensor (R10) and the eighth backrest sensor (R16) are configured to monitor the middle thoracic region of the user, the fourth backrest sensor (R12) is configured to monitor the central lumbar region of the user and the fifth backrest sensor (R13) is configured to monitor the upper-central thoracic region of the user.

9. The system (1) of any one of the preceding claims, wherein the 16 pressure sensors (A1-8, R9-16) are selected from resistive pressure sensors, capacitive pressure sensors and a combination of both.

10. The system (1) of claim 9, wherein the 16 pressure sensors (A1-8, R9-16) are resistive pressure sensors.

11. The system (1) of any one of the preceding claims, wherein the seat portion (5) and the backrest portion (6) are symmetrical with respect to an XZ plane (19) located in correspondence with the coordinate origin (20).
